# EUROPÄISCHE PATENTANMELDUNG

(11) **EP 0 749 723 A1**
(43) Veröffentlichungstag der Anmeldung: **27.12.1996**
(21) Anmeldenummer: 95250150.0
(22) Anmeldetag: 23.06.1995
(51) Int. Cl.: A61B 8/12

(54) **Intestinal-Ultraschallsonde zur transintestinalen Diagnose bei Vögeln, Reptilien und/oder kleinen Säugetieren**

(71) Anmelder: Arno Schnorrenberg Chirurgiemechanik, D-13086 Berlin (DE)
(72) Erfinder: Schnorrenberg, Arno, Dipl.-Ing., D-13086 Berlin (DE)
(74) Vertreter: Neumann, Günter

(57) **Zusammenfassung**

Die Erfindung bezieht sich auf eine Intestinal-Ultraschallsonde zur transintestinalen Diagnose bei Vögeln, Reptilien und/oder kleinen Säugetieren.
Sie besteht aus einem schlauchförmigen flexiblen Halte- und Einführungsteil (1) in dessen distales Ende ein Ultraschallkopf (2) integriert ist, der eine parallel zu seiner Längsachse verlaufende Abflachung aufweist, auf der in einer rechteckigen Grundform Piezokristalle aufgebracht sind und bei der im Halte- und Einführungsteil (1) ein Katheter (3) für einen Führungsdraht und/oder eine Spülflüssigkeit zur transintestinalen Anwendung der Sonde vorgesehen ist.

Die Erfindung ermöglicht eine tierschutzkonforme transintestinale Diagnose bei Vögeln, insbesondere kleiner Vogelarten, bei Reptilien und kleinen Säugetieren. Am lebenden, nicht-narkotisierten Tier können in einem streß- und risikoarmen Untersuchungsgang Bildinformationen über Größe, Form und Echostruktur vieler viszeraler Organe bereitgestellt werden.
Auf einfache Weise und kostengünstig können die Geschlechter monomorpher Vogelarten sicher identifiziert werden. Die Erfindung ist ein vorteilhaftes Hilfsmittel zur Lösung reproduktionsphysiologischer Problemstellungen in der Zucht und Produktion von Nutzgeflügel.

## Beschreibung

Die Erfindung bezieht sich auf eine Intestinal-Ultraschallsonde zur transintestinalen Diagnose bei Vögeln, Reptilien und/oder kleinen Säugetieren.

Eine Intestinal-Ultraschallsonde zur transintestinalen Diagnostik bei kleinen Säugetieren (Schafe) ist aus der DE-OS 3432281 bekannt.
Zur Bestimmung der Geschlechter bei monomorphen Vogelarten sowie zur Identifikation und Darstellung pathologischer Veränderungen viszeraler Organe, wie Keimdrüsen, Eileiter bzw. Uterus, Leber, Milz und Niere sowie großer Blutgefäße bei Vögeln, Reptilien und/oder kleinen Säugetieren ist die bekannte Sonde nicht geeignet.

Bereits seit Mitte der 70er Jahre gewinnt die Ultraschalldiagnostik auch in der Veterinärmedizin zunehmend an Bedeutung. In der Praxis und in der wissenschaftlichen Literatur wurden jedoch Sonographieuntersuchungen für Vogelarten lange Zeit als nicht durchführbar angesehen. Als wesentliche Ursache hierfür werden die anatomischen Besonderheiten von Vögeln, insbesondere das bei dieser Tierart vorhandene Luftsacksystem angeführt.

In jüngster Zeit ist jedoch auch am Vogel die Ultraschalldiagnostik zur Anwendung gekommen, und zwar als transkutane Ultraschalluntersuchung. Mit der hierfür eingesetzten Ultraschalltechnik sind jedoch eine Reihe von Nachteilen verbunden. So wird in der wissenschaftlichen Literatur insbesondere auf die durch die geringe Ankopplungsfläche der Gerätetechnik und die durch die Abdominalluftsäcke bedingten Grenzen der transkutanen Ultrasonographietechnik hingewiesen (Silvermann, S.: New frontiers in imaging avian and zoologie patients. Procedings 1. International Conference of Zoological and Avian Medicine, 1987, p. 351-352); McMillan, M.C.: Imaging of avian urogenital disorders in Journal of the Association of Avian Veterinarians 2, 1988, Seiten 74-82; Riedel, U.: Die Ultraschalluntersuchung des Vogels am Beispiel der Brieftaube, PH. D. Dissertation, 1992, Justus-Liebig-Universität Gießen. Vor allem durch das beim Vogel vorhandene Luftsacksystem, das kompakte Darmkonvolut und die subserösen Fetteinlagerungen wird die transkutane Ausbreitung der Schallwellen stark gestört.

Mit der transkutanen Ultrasonographietechnik werden zwar für die außerhalb der Abdominalluftsäcke gelegenen inneren Organe durchaus befriedigende Resultate erzielt, jedoch wird übereinstimmend insbesondere darauf verwiesen, daß zum Beispiel die Vogelniere infolge der genannten Grundschwierigkeiten nicht darstellbar ist. Desweiteren wird in der Literatur die prinzipielle sonographische Nichtdarstellbarkeit der unmittelbar dem Beckenskelett aufliegenden Vogelgonaden und anderen Viszera hervorgehoben.

Bei Reptilien lassen insbesondere die kaudal reichenden Lungensäcke sowie das teilweise sehr stark verhornte Integumentum eine sonographische Feindarstellung der inneren Organe nicht zu.
Bei kleinen Säugetieren ist diese Technik mit dem Nachteil verbunden, daß eine Vorbehandlung der Tiere, beispielsweise das Entfernen des Haarkleides erforderlich ist.

Mit der bisher bekannten transkutanen Ultrasonographietechnik ist deshalb bei Vögeln, Reptilien und kleinen Säugetieren auch nur sehr eingeschränkt und mit problematischer diagnostischer Wertigkeit und Reproduzierbarkeit die Topographie der inneren Organe dieser Tierarten feststellbar.

In Anbetracht dieser Nachteile liegt der Erfindung die Aufgabe zugrunde, bei Überwindung der Grenzen der transkutanen Ultrasonographietechnik ein Gerät zur transintestinalen sonographischen Diagnose bereitzustellen, das insbesondere die Identifikation und Darstellung der Struktur sowie der physiologischen und pathologischen Veränderungen innerer Organe bei Vögeln, Reptilien und/oder kleinen Säugetieren in zweidimensionaler Bilddarstellung ermöglicht.

Erfindungsgemäß wird die Aufgabe durch eine Intestinal-Ultraschallsonde mit den kennzeichnenden Merkmalen nach Anspruch 1 gelöst. Weitere vorteilhafte Ausgestaltungen der Erfindung sowie die erfindungsgemäße Verwendung einer Intestinal-Ultraschallsonde ergeben sich aus den Ansprüchen 2 bis 9.

Die erfindungsgemäße Lösung hat gegenüber dem bekannten Stand der Technik, insbesondere der kostenaufwendigen Endoskopietechnik erhebliche Vorteile.
Insbesondere ermöglicht die Erfindung erstmals eine tierschutzkonforme transintestinale Diagnose bei Vögeln, insbesondere kleiner Vogelarten, bei Reptilien und kleinen Säugetieren. Durch die im Darmlumen dieser Tierarten via Kloake bzw. Rectum einführbare erfindungsgemäßen Sonde können am lebenden, nicht-narkotisierten Tier in einem streß- und risikoarmen Untersuchungsgang Bildinformationen über Größe, Form und Echostruktur vieler viszeraler Organe, wie Milz, Niere einschließlich Nierengefäße und Harnleiter sowie der Bursa fabrici bereitgestellt werden.
Vor allem soll darauf hingewiesen werden, daß mit der erfindungsgemäßen Intestinal-Ultraschallsonde auf einfache Weise und kostengünstig die Geschlechter monomorpher Vogelarten anhand der Lage, Form und echogenen Struktur der Keimdrüsen sicher identifiziert werden können. Darüber hinaus lassen sich der Funktionszustand der Gonaden und der Eileiter beurteilen sowie pathologische Veränderungen der Samen- und Harnleiter feststellen.

Mit der einfach handhabbaren erfindungsgemäßen Sonde kann sozusagen vor Ort und erstmals nicht-invasiv der Verlauf der gesamten Phase der Eibildung visuell dargestellt werden. Damit bietet sich die Erfindung zugleich als ein vorteilhaftes Hilfsmittel zur Lösung reproduktionsphysiologischer Problemstellungen in der Zucht und Produktion von Nutzgeflügel an. So kann die Zuchtauswahl von Nutzgeflügel, beispielsweise von Hühnervögeln, in Abhängigkeit von den Erkenntnissen der visuellen Darstellung der Eischalenbildung im Legeapparat sowie der Eierstock- und Hodenaktivität vorgenommen werden.

Im folgenden sollen die Erfindung und deren Verwendung anhand von Zeichnungen näher erläutert werden. Es zeigen
- Fig.1: die Schnittdarstellung einer Intestinal-Ultraschallsonde
- Fig.2: in schematischer Darstellung Ausführungsformen des Ultraschallkopfes.

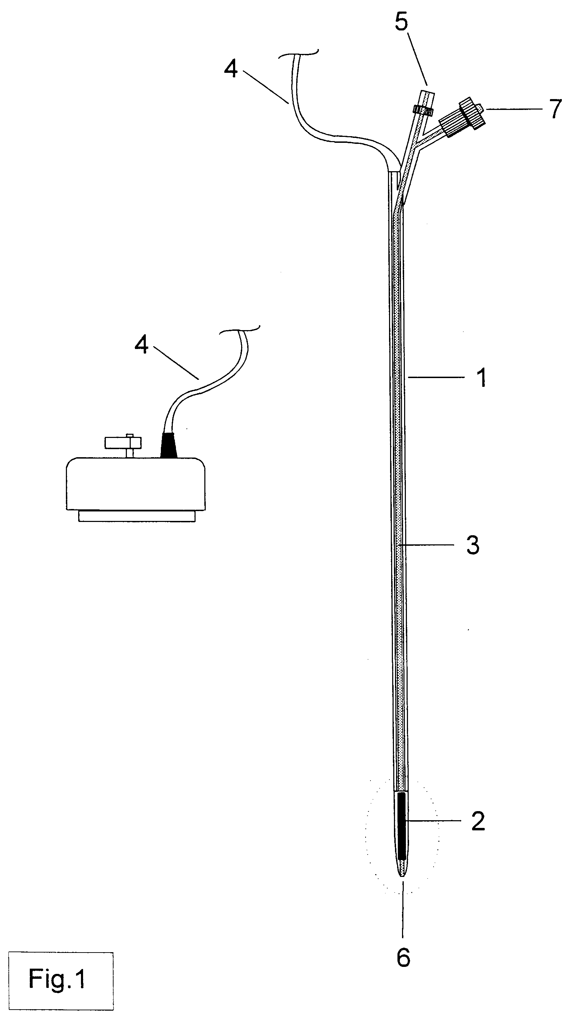

In Fig. 1 ist eine Intestinal-Ultraschallsonde im Längsschnitt dargestellt.
Die Sonde besteht aus einem schlauchförmigen flexiblen Halte- und Einführungsteil 1, der einen äußeren Durchmesser bis max. 5 mm aufweist. An seinem Ende ist der Ultraschallkopf 2 angeordnet, dessen Piezokristalle monoplan in Form eines deutlich zu erkennenden Rechteckes angeordnet sind. Sie können auch biplan oder curved-linear aufgebracht sein. Der vordere Bereich des Schallkopfes 2 kann demnach über seine Längsachse nach oben ausgebogen sein.
Der Schallkopf 2 ist für einen Frequenzbereich von 5 bis 15 MHz, vorzugsweise für 10 MHz, vorgesehen, weist 50 bis ca. 200 Piezokristalle auf und ist mit einer ultraschalldurchlässigen Schutzschicht abgedeckt. Der Schallkopf 2 kann bis zu 20 mm lang sein und ist etwa 3 mm breit.
Die auf dem Schallkopf 2 angeordneten Piezokristalle erzeugen mittels elektronischer Schallstrahlsteuerung und -formung ein zweidimensionales Bild.
Im Halte- und Einführungsteil 1 ist der Katheter 3 angeordnet, der einen inneren Durchmesser bis zu 1 mm, vorzugsweise von 0,9 mm aufweist.

Über die Zuleitung 4 ist die erfindungsgemäße Sonde mit einem angepaßten handelsüblichen tragbaren Ultrasonographiegerät verbunden.
Die zur Daten- und/oder Energieübertragung zu den Piezokristallen erforderlichen Verbindungsleitungen sind koaxial im Raum zwischen Katheter 3 und Halte- und Einführungsteil 1 angeordnet. Sie können auch direkt in die Wandung des Halte- und Einführungsteils 1 integriert sein.
Der Katheter 3 ist zur Aufnahme eines Führungsdrahtes - hier nicht dargestellt - vorgesehen. Er wird über die dafür eingerichtete Öffnung 6 in den je nach Art und Größe des zu untersuchenden Tieres bis zu 30 cm langen Halte- und Einführungsteil 1 eingebracht und kann durch die Austrittsöffnung 5 wieder aus der Sonde entfernt werden.
Erforderlichenfalls dient der Katheter 3 auch der Zuführung eines Spülmittels zur Spülung des Darmes. Zu diesem Zweck ist auch die Spüleinrichtung 7 am Halte- und Einführungsteil 1 vorgesehen.

Bei der Anwendung der Sonde, beispielsweise bei einem Kleinvogel, wird zunächst der Führungsdraht via Kloake auf an sich bekannte Weise unter Sichtkontrolle endoskopisch in den Darm vorgelegt. Das aus dem Tier hinausragende Ende des Führungsdrahtes wird in die Öffnung 6 des Halte- und Einführungsteils eingebracht und die Sonde daraufhin über den Führungsdraht in den Darm vorgeschoben. Es versteht sich, daß das mit der gebotenen Vorsicht zu erfolgen hat, um den Führungsdraht und die Sonde sicher - und ohne den nicht-narkotisierten Vogel zu verletzen - an den teilweise bestehenden Darmkloakenfalten vorbei einzuführen. Der flexible und schlauchförmige Halte- und Einführungsteil 1 gestattet es, die erfindungsgemäße Sonde langsam im Darmlumen nunmehr unter sonographischer Sichtkontrolle vorzuschieben und über dem entsprechenden Organ den Ultraschallkopf 2 zu positionieren. Der Führungsdraht wird anschließend, wie bereits beschrieben, über die Austrittsöffnung 5 wieder entfernt.
Die Zuführung von Flüssigkeit über Katheter 3 kann auch zur Ausbildung einer intestinalen Vorlaufstrecke genutzt werden. Das hat den Vorteil, daß die Spitze der Sonde in einem Wasserpolster gleitend im Darm vorgebracht und sensibel positioniert werden kann. Die Ausbildung eines Flüssigkeitspolsters im Bereich des Schallkopfes 2 wirkt sich darüber hinaus vorteilhaft auf die Beseitigung von Schallartefakten aus, ermöglicht einen besseren Focusbereich für schallkopfnahe Strukturen und optimiert insofern die Bilddarstellung.
Als besonders vorteilhaft erweisen sich dabei die auf dem Halte- und Einführungsteil 1 vorgesehenen Längs- und Quermarkierungen. Sie ermöglichen in jedem Stadium der Untersuchung eine genaue Lagebestimmung des Schallkopfes 2. Darüber hinaus dienen sie der biometrischen Bestimmung anatomischer und pathologischer Strukturen und der Lagebeziehung der inneren Organe des zu untersuchenden Tieres. Die dabei gewonnnenen Daten sind außerdem unverzichtbar für das Erkennen und Bestimmen von Veränderungen auf der Grundlage von Folgeuntersuchungen.

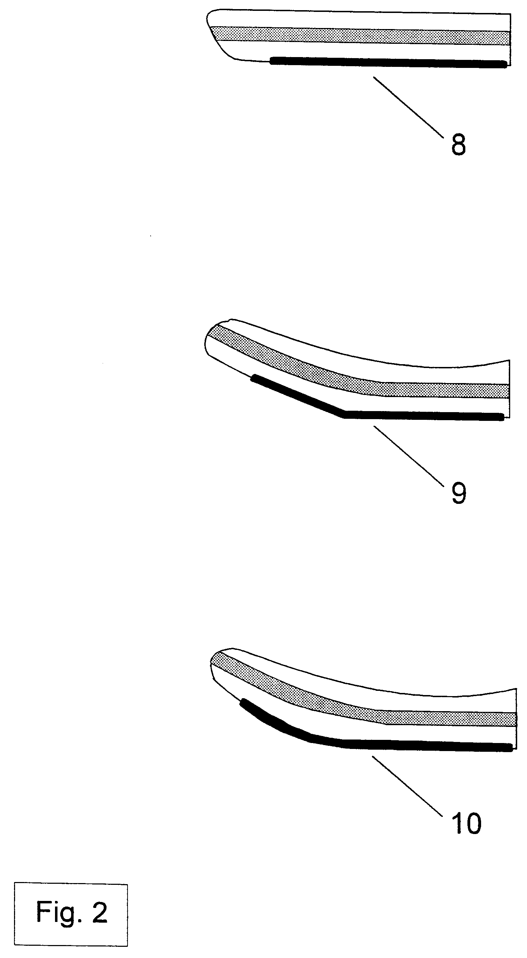

Wie aus Fig. 2 zu erkennen, können die hier einzeln nicht zu sehenden, akustisch gegeneinander isolierten, Piezokristalle monoplan 8, biplan 9 oder curved-linear 10 auf einer hier nicht dargestellten Anpassungs- und Dämmschicht angeordnet sein.

Die Anordnung der Piezokristalle in monoplaner, biplaner oder curved-linearer Form parallel zur Längsachse des Schallkopfes 2 hat den wesentlichen Vorzug, daß bei dem genannten Frequenzbereich eine Darstellungstiefe bis zu 70 mm erreicht wird, da die Schallwellen im wesentlichen rechtwinklig auf die durch die Spülflüssigkeit gereinigte Darmwand und die an der Darmwand angrenzenden Vizeral-Organe treffen, ohne durch das Luftsacksystem Behinderungen zu erfahren, die die Darstellung der inneren Organe wesentlich einschränken, wenn nicht sogar unmöglich machen. Insofern unterscheidet sich die erfinderische Sonde grundlegend von Sonden mit an der Spitze angebrachten Piezokristallen, die mit der Schallabstrahl- und Schallempfangsseite in Vorausrichtung zeigen bzw. am Gesamtumfang der Sonde angebrachten und 360°-Bilder erzeugenden Piezokristallen. Bei derartigen Anordnungen der Piezokristalle werden die Schallwellen durch im Darm befindliche Ingestamassen und die durch das am Darm ventral anliegende Luftsacksytem bedingten Artdefakten so stark modifiziert und behindert, daß es zu Schallauslöschungen, -überlagerungen und -verstärkungen kommt, die eine völlig gestörte Bilddarstellung zur Folge haben und eine Begutachtung der zu untersuchenden Organe nicht ermöglichen.

Für die Auswahl der Materialien für den Halte- und Einführungsteil, den Katheter, den Führungsdraht sowie die Anpassungs- und Dämmschicht für die Piezokristalle finden die Erfahrungen Anwendung, die bisher bei Endoskopen oder mechanischen Ultraschallsonden gesammelt wurden. Für den Halte- und Einführungsteil sowie den Katheter kommen insbesondere flexible Kunststoffe in Frage.

### Bezugszeichenaufstellung

- 1: Halte- und Einführungsteil
- 2: Schallkopf
- 3: Katheter
- 4: Zuleitung
- 5: verschließbare Austrittsöffnung
- 6: Öffnung
- 7: Spülvorrichtung
- 8: monoplane Anordnung der Piezokristalle
- 9: biplane Anordnung der Piezokristalle
- 10: curved-lineare Anordnung der Piezokristalle

## Patentansprüche

1. Intestinal-Ultraschallsonde zur transintestinalen Diagnose bei Vögeln, Reptilien und/oder kleinen Säugetieren, **dadurch gekennzeichnet,** daß sie
- aus einem schlauchförmigen flexiblen Halte- und Einführungsteil (1) besteht,
- am distalen Ende des Halte- und Einführungsteils (1) ein Ultraschallkopf (2) integriert ist, der eine parallel zu seiner Längsachse verlaufende Abflachung aufweist, auf der in einer rechteckigen Grundform Piezokristalle aufgebracht sind und
- im Halte- und Einführungsteil (1) ein Katheter (3) für einen Führungsdraht und/oder eine Spülflüssigkeit zur transintestinalen Anwendung der Sonde vorgesehen ist.

2. Sonde nach Anspruch 1, **dadurch gekennzeichnet,** daß der Ultraschallkopf (2) mit 50 bis ca. 200, vorzugsweise 100 Piezokristallen bestückt ist und eine Länge bis zu 20 mm, vorzugsweise 15 mm, sowie eine Breite von etwa 3 mm aufweist und mit einer üblichen ultraschalldurchlässigen Schutzschicht versehen ist.

3. Sonde nach Anspruch 1 oder 2, **dadurch gekennzeichnet,** daß die Piezokristalle monoplan (8), biplan (9) oder curved-linear (10) auf einer Anpassungs- und Dämpfungsschicht angeordnet sind.

4. Sonde nach einem der Ansprüche 1 bis 3, **dadurch gekennzeichnet,** daß es sich um Piezokristalle für einem Frequenzbereich von 5 bis 15 MHz, vorzugsweise 10 MHz, handelt.

5. Sonde nach Anspruch 1, **dadurch gekennzeichnet,** daß der Katheter (3) einen inneren Durchmesser bis 1 mm, vorzugsweise von 0,9 mm aufweist.

6. Sonde nach Anspruch 1, **dadurch gekennzeichnet,** daß zwischen dem schlauchförmigen flexiblen Halte- und Einführungsteil (1) und dem Katheter (3) oder direkt in der Wandung des Halte- und Einführungsteils (1) Verbindungsleitungen zur Daten- und/oder Energieübertragung zwischen den Piezokristallen des Ultraschallkopfes (2) und einem am anderen Ende über die Zuleitung 4 verbundenen externen Anschlußgerät angeordnet sind.

7. Sonde nach Anspruch 1, **dadurch gekennzeichnet,** daß das schlauchförmige flexible Halte- und Einführungsteil (1) einen äußeren Durchmesser bis zu 5 mm, vorzugsweise 3 mm, besitzt sowie Quer- und Längsmarkierungen aufweist.

8. Sonde nach einem der Ansprüche 1 bis 7, **dadurch gekennzeichnet,** daß am vom Tier abgewandten Ende der Sonde eine Austrittsöffnung (5) für das Entfernen des Führungsdrahtes und eine Vorrichtung (7) für das Einbringen einer externen Spülflüssigkeit vorgesehen sind.

9. Verwendung einer Intestinal-Ultraschallsonde zur transintestinalen Diagnose bei Vögeln, Reptilien und/oder kleinen Säugetieren, bestehend aus einem schlauchförmigen flexiblen Halte- und Einführungsteil (1) mit darauf angebrachten Längs- und Quermarkierungen, das einen äußeren Durchmesser bis zu 5 mm, vorzugsweise 3 mm, aufweist, einem darin am distalen Ende integrierten Ultraschallkopf (2), der eine parallel zu seiner Längsachse verlaufende Abflachung aufweist, auf der in einer rechteckigen Grundform Piezokristalle monoplan, biplan oder curved-linear aufgebracht sind und der bei einer Breite von etwa 3 mm eine Länge bis zu 20 mm, vorzugsweise 15 mm, aufweist und aus 50 bis ca. 200, vorzugsweise 100 Piezokristallen für eine Frequenz im Bereich von 5 MHz bis 15 MHz, vorzugsweise 10 MHz besteht, und einem im Halte- und Einführungsteil (1) angeordneten Katheter (3) mit einem inneren Durchmesser bis zu 1 mm, vorzugsweise von 0,8 mm, wobei zwischen dem schlauchförmigen flexiblen Halte- und Einführungsteil (1) und dem Katheter (3) oder direkt in der Wandung des Halte- und Einführungsteils (1) Verbindungsleitungen zur Daten- und/oder Energieübertragung zwischen den Piezokristallen des Ultraschallkopfes (2) und einem am anderen Ende vorgesehenen externen Anschlußgerät angeordnet sind.
